# EUROPEAN PATENT SPECIFICATION

(11) **EP 2 331 074 B1**
(45) Date of publication and mention of the grant of the patent: **11.11.2020**
(21) Application number: 09814882.8
(22) Date of filing: 16.09.2009
(51) Int. Cl.: A61K 9/16, A61K 9/20, A61K 31/277

(54) **GRANULATES, PROCESS FOR PREPARING THEM AND PHARMACEUTICAL PRODUCTS CONTAINING THEM**
GRANULAT, VERFAHREN ZU SEINER HERSTELLUNG UND PHARMAZEUTISCHE PRODUKTE DAMIT
GRANULÉS, LEUR PROCÉDÉ DE PRÉPARATION ET PRODUITS PHARMACEUTIQUES LES CONTENANT

(30) Priority: 17.09.2008 US 97667 P
(43) Date of publication of application: 15.06.2011
(73) Proprietor: Mylan Inc., Morgantown, WV 26505 (US)
(72) Inventor: LI, Boyong, Morgantown West Virginia 26508 (US); REYNOLDS, Thomas D., Morgantown West Virginia 26505 (US)
(74) Representative: Elkington and Fife LLP
(86) International application number: PCT/US2009/005153
(87) International publication number: WO 2010/033179

(56) References cited:
- EP-A2- 1 889 610
- WO-A1-01/42221
- WO-A1-2004/100944
- WO-A1-2004/110431
- WO-A1-2004/110431
- WO-A1-2007/011349
- WO-A2-2008/101723
- WO-A2-2008/104852
- US-A1- 2006 222 707
- US-A1- 2008 161 404
- US-A1- 2008 161 404

## Description

### FIELD OF THE INVENTION

The present invention relates to granules having a core containing an active pharmaceutical ingredient that itself has poor aqueous solubility where the active pharmaceutical ingredient is intimately associated with one or more hydrophilic polymers. The granules are useful in the manufacture of pharmaceutical compositions, as exemplified by formulations of bicalutamide.

### BACKGROUND OF THE INVENTION

The aqueous solubility of an active pharmaceutical ingredient ("API") influences both the bioavailability of the drug and the rate at which the API can be released from a formulated product. The rate of dissolution of an API from a formulation can place an upper limit on the rate of absorption of the API in a person to whom the product is administered. Many active pharmaceutical ingredients have poor aqueous solubility and low bioavailability. One method that has been used to improve the dissolution of API's is to reduce the particles size of the active ingredient, which increases the surface area of the active ingredient and may result in an increased rate of dissolution. This approach is limited by the particle size that can be achieved and by poor bulk flow and handling characteristics of finely powdered active pharmaceutical ingredients, which often require special isolation and handling procedures due to the toxicological nature of many API's. One method for improving the dissolution rate involves spray drying a solution of the API and hydrophilic polymers. (See, Marc Hugo, et al.: Dissolution rate of poorly soluble fenofebrate can be improved by solid dispersion in hydrophilic polymers using spray drying. AAPS Annual Meeting and Exposition, October 2, 2006. San Antonio, Texas). This approach is limited by the resulting fine powder material that has poor bulk flow and handling characteristics and the need for extra processing steps to make solid dosage forms such as tablets and capsules. Another process for enhancing dissolution involves kneading a mixture of an API with polyvinyl pyrrolidone dispersed in water, drying the paste, to form a powder. The powder is then screened and compressed into tablets. (See, Aftab Modi and Pralhad Tayade: Enhancement of dissolution profile by solid dispersion (kneading) technique. AAPS PharmSciTech 2006. 7(3), Article 68). The kneading process is limited by the special difficulties involved in drying the paste material.

The standard one-step granulation process commonly used in the pharmaceutical industry produces granules by adding a solution or mixture of a drug and an excipient, such as a binder, to a solid mixture of other excipients. When the drug substance has poor aqueous solubility, this process is found to be unsuitable because the granules formed include large agglomerates and the time duration of the granulation process has a sharp endpoint. In addition, although tablets made from a one-step wet granulation process show acceptable dissolution characteristics, these tablets are observed to erode unevenly during dissolution testing. These observed processing and dissolution characteristics are believed to be related to a non-uniform distribution of the binder, such as povidone, which acts as a wetting agent and the inadequate wetting of the drug substance during granulation. When the drug substance has poor aqueous solubility, the standard wet granulation process also results in inadequate and uneven contact between the drug and hydrophilic polymer.

Many API's have low aqueous solubility. An example of an API with low aqueous solubility is bicalutamide, which has a water solubility of 5 mg/1000 ml at 37°C. Bicalutamide is the common name for the compound *N*-[4-cyano-3-(trifluoromethyl)phenyl]-3-(4-fluorophenyl)sulfonyl-2-hydroxy-2-methyl-propanamide, which is also known as 4'-cyano-3-((4-fluorophenyl) sulfonyl)-2-hydroxy-2-methyl-3'-(trifluoromethyl)propionanilide. The structure of bicalutamide is shown in formula (I) below:

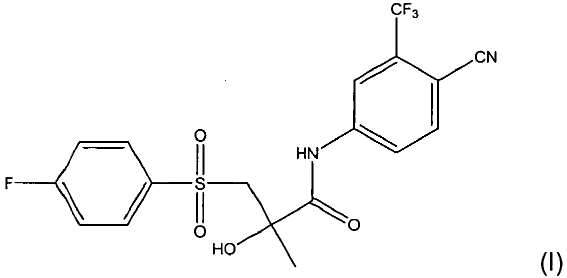

Bicalutamide is an oral non-steroidal anti-androgen used for the treatment of prostate cancer and hirsutism.

### SUMMARY OF THE INVENTION

To address these process and dissolution issues, the process described herein, termed "reverse wet granulation" was developed. In the process, the API is intimately mixed with a solution or suspension of a hydrophilic polymer to form a drug-polymer slurry. Granules can then be formed by incorporating a mixture of other dry excipients into the drug-polymer slurry. The granules formed comprises a core containing an active pharmaceutical ingredient that itself has poor aqueous solubility where the active pharmaceutical ingredient is intimately associated with one or more hydrophilic polymers. The term granulate, when used as a noun, refers to group of granules. Granules produced by this process, after milling, have good flow and handling characteristics like those produced with the commonly used one-step process. However, tablets formed from these granules erode more uniformly during dissolution testing.

According to one aspect of the invention, granules for use in a pharmaceutical composition comprises a core comprising at least one active pharmaceutical ingredient intimately associated with at least one hydrophilic polymer, where the active pharmaceutical ingredient has a solubility in water of less than about 1 mg/ml.

According to another aspect of the invention, the granule further comprises at least one excipient selected from the group consisting of diluents, disintegrants, binders, wetting agents, lubricants, glidants, coloring agents and flavoring agents.

According to yet another aspect of the invention, a pharmaceutical dosage form comprises a granule which comprises a core with at least one active pharmaceutical ingredient intimately associated with at least one hydrophilic polymer, where the active pharmaceutical ingredient has a solubility in water of less than about 1 mg/ml.

According to still another aspect of the invention, a process for making a granulate comprises the steps of:
(a) dissolving or suspending at least one hydrophilic polymer in a solvent to form a solution or suspension, respectively,
(b) combining the solution formed in step (a) with an active pharmaceutical ingredient having a solubility in water of less than about 1 mg/ml to form a mixture and blending the mixture to form a slurry,
(c) combining the mixture formed in step (b) with an excipient or a mixture of excipients to form a wet granulate, and
(d) drying the wet granulate to obtain a dry granulate where the wet granulate formed in step (c) comprises a core containing an active pharmaceutical ingredient that itself has poor aqueous solubility where the active pharmaceutical ingredient is intimately associated with one or more hydrophilic polymers.

According to another aspect of the invention, the process for making a granulate further comprises the step of milling the dry granulate to obtain a modified dry granulate having a desired particle size distribution.

According to yet another aspect of the invention, a process for making a pharmaceutical tablet comprises compressing a granulate comprising at least one active pharmaceutical ingredient intimately associated with at least one hydrophilic polymer, where the active pharmaceutical ingredient has a solubility in water of less than about 1 mg/ml, into tablets.

According to still another aspect of the invention, the granulate is a composition produced by a process comprising the steps of:
(a) forming a mixture core by (1) dissolving or suspending at least one hydrophilic polymer in a solvent to form a solution or suspension, respectively, and (2) combining the solution or suspension formed in step (1) with an active pharmaceutical ingredient having a solubility in water of less than about 1 mg/ml to form a mixture and blending the mixture to form a slurry,
(b) combining the mixture formed in step (a) with an excipient or a mixture of excipients to form a wet granulate, and
(c) drying the wet granulate to obtain a dry granulate.

### BRIEF DESCRIPTION OF THE DRAWING

Figure 1 is a representation of the granule of the invention.
Figure 2 depicts a process for manufacturing the granulate by reverse wet granulation.

### DETAILED DESCRIPTION OF THE INVENTION

The present invention provides granules comprising having an active pharmaceutical ingredient having poor aqueous solubility and methods for making such granules. The granules are useful for making oral solid dosage forms, for example capsules and compressed tablets, in a variety of shapes and sizes. The advantages of the present inventive composition and method are notable with active pharmaceutical ingredients that have poor aqueous solubility.

In this application, the term "active pharmaceutical ingredient, which has poor aqueous solubility" or "active pharmaceutical ingredient having poor aqueous solubility" means an API or drug having a solubility in water of less than about 1 mg/mL, i.e., the compound is water insoluble (<0.1 mg/mL) or very slightly soluble (0.1 - 1.0 mg/mL), according to the USP definition of solubility. Examples of an "active pharmaceutical ingredient, which has poor aqueous solubility" or an "active pharmaceutical ingredient having poor aqueous solubility" include anastrozole, aripiprazole, atorvastatin, bicalutamide, candesartan, celecoxib, dutasteride, ezetimibe, fenofibrate, glyburide, meloxicam, oxcarbazepine, raloxifene, rifaximine, rofecoxib, simvastatin, and valdecoxib. In a preferred embodiment, the active pharmaceutical ingredient is bicalutamide. The granules described herein can be used with racemic mixtures of an active ingredient having poor aqueous solubility or with individual isomers of such active ingredient. The granules can comprise one or more active ingredients where at least one of the active ingredients has poor aqueous solubility.

In the granules of the invention, the active pharmaceutical ingredient having poor aqueous solubility and the one or more pharmaceutically acceptable hydrophilic polymers are intimately associated or are in intimate association. The term "intimately associated" or "intimate association" refers to a state produced by a process comprising mixing the API and a solution of the one or more pharmaceutically acceptable hydrophilic polymers to form a mixture in the form of a slurry. The API and the one or more pharmaceutically acceptable hydrophilic polymers are intimately associated or in intimate association, with the hydrophilic polymer providing a coating on particles of the API. Granules are formed by mixing one or more excipients with the intimate association of the API and the at least one hydrophilic polymer. This subunit of the granule is termed the drug dispersion.

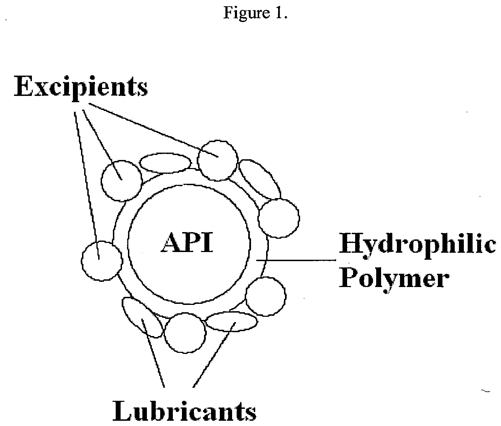

Granules of this invention are represented in Figure 1. The granule comprises a core to which one or more excipients are in contact or attached. The core comprises at least one API and the one or more pharmaceutically acceptable hydrophilic polymers, where the one or more pharmaceutically acceptable hydrophilic polymers coat particles of the API. One or more excipients are in contact or attached to the core. The granule may also include additional excipients, as exemplified by one or more lubricants, which are added to the granule in a further processing step.

The core of the granule, where the API and the one or more pharmaceutically acceptable hydrophilic polymers are in intimate association, achieves a consistency and stable adherence between the API and the at least one hydrophilic polymer. As a result of the intimate association between the API and the at least one hydrophilic polymer, pharmaceutical compositions produced using granules of the invention have more uniform wetting and dissolution.

Examples of pharmaceutically acceptable hydrophilic polymers include polyvinyl pyrrolidone (also known as PVP or povidone), hydroxypropyl methylcellulose, hydroxypropyl cellulose, polyethylene glycol, hydroxyethyl cellulose, polyethylene oxide, carbomer, polyvinyl alcohol, and/or mixtures thereof. In one particular embodiment, the hydrophilic polymer is povidone.

The granule may further comprise at least one excipient in addition to the one or more pharmaceutically acceptable hydrophilic polymers. Such excipients include diluents, disintegrants, binders, wetting agents, lubricants, glidants, coloring agents and flavoring agents. Examples of such excipients are well known to one skilled in the art. See for example Rowe, et al., Handbook of Pharmaceutical Excipients, 5th Edition, which is hereby incorporated by reference in its entirety. In one embodiment, the at least one excipient comprises at least one diluent and at least one disintegrant. Examples of diluents include lactose monohydrate, microcrystalline cellulose, calcium phosphate dibasic, sucrose, mannitol, starch, pregelatinized starch, lactose, sorbitol, glucose, fructose, galactose, maltose, isomaltose, aluminum oxide, bentonite, powdered cellulose, kaolin, magnesium carbonate, saponite, and mixtures thereof. In one particular embodiment, the diluent is lactose monohydrate. Examples of disintegrants include crospovidone, croscarmellose sodium, sodium starch glycolate, and mixtures thereof. In an embodiment the disintegrant is sodium starch glycolate. Examples of lubricants include magnesium stearate, sodium lauryl sulfate, colloidal silicon dioxide, calcium stearate, magnesium lauryl sulfate, potassium benzoate, sodium benzoate, talc, zinc stearate, sodium stearyl fumarate and mixtures thereof. In one embodiment, the lubricant is a mixture of magnesium stearate and sodium lauryl sulfate. In another embodiment, the lubricant is colloidal silicon dioxide. In yet another embodiment, both colloidal silicon dioxide and a mixture of magnesium stearate and sodium lauryl sulfate are used together.

In one embodiment, the granule comprises bicalutamide as the active pharmaceutical ingredient and povidone as the at least one hydrophilic polymer. In another embodiment, the granule comprises bicalutamide as the active pharmaceutical ingredient, povidone as the at least one hydrophilic polymer, and lactose monohydrate and sodium starch glycolate as excipients. In still another embodiment, the granule comprises bicalutamide as the active pharmaceutical ingredient, povidone as the at least one hydrophilic polymer, and lactose monohydrate, sodium starch glycolate and povidone as excipients. In a further embodiment, the granule comprises bicalutamide as the active pharmaceutical ingredient, povidone as the at least one hydrophilic polymer, and lactose monohydrate, sodium starch glycolate and povidone as excipients, and colloidal silicon dioxide and a mixture of magnesium stearate and sodium lauryl sulfate as lubricants. In another embodiment, the granule further comprises at least one hydrophilic polymer that is not intimately associated with the active ingredient. The weight ratio of the API to the at least one hydrophilic polymer in the granule is preferably at least 1:10, more preferably at least 1:5, and even more preferably at least 1:1, and is preferably less than 50:1, more preferably less than 20:1 and even more preferably less than 10:1.

The granule described in the various embodiments above can be used in pharmaceutical dosage forms, such as tablets and capsules. In one embodiment, the dosage form is a tablet and the API comprises bicalutamide. In another embodiment, the dosage form is a tablet, the API comprises bicalutamide and the at least one hydrophilic polymer is povidone. In yet another embodiment, the dosage form comprises granules which comprise at least one diluent, at least one disintegrant and at least one lubricant. In still another embodiment, the dosage form comprise granules which comprise lactose monohydrate, as the diluent, sodium starch glycolate as the disintegrant and a mixture of magnesium stearate/sodium lauryl sulfate as the lubricant. In a further embodiment, the dosage form comprise granules which comprise lactose monohydrate, as the diluent, sodium starch glycolate as the disintegrant and both colloidal silicon dioxide and a mixture of magnesium stearate/sodium lauryl sulfate as the lubricant.

The solid pharmaceutical formulations, e.g., tablets and capsules, of the present invention can display dissolution properties that can be adjusted to obtain a desired profile by altering the specific excipients used within the capsules or tablets as well as by altering the nature and/or quantity of a coating on the tablets. Altering the nature and/or quantity of the excipients in a tablet or capsule and/or a coating on a tablet to obtain a desired release rate can be performed using methods known to one of ordinary skill in the art. In one embodiment, minitablets comprising the granule described herein may be contained within capsules. A capsule may contain minitablets having essentially a uniform release rate or may contain minitablets having different release rates. Methods of adjusting the overall release rate of an active ingredient from a capsule using a plurality of minitablets having different individual release rates are known to one of ordinary skill in the art.

In one embodiment, the pharmaceutical dosage form is a tablet comprising granules which comprise bicalutamide as the API, wherein the amount of bicalutamide dissolves in the time listed below when tested under conditions described as the USP apparatus II (paddle) test, using 1000 ml of a 1% aqueous solution of sodium lauryl sulfate at 37° C with the paddle apparatus rotating at 50 rpm.

| approximate % dissolved | approximate time (minutes) |
|---|---|
| 30 | 5 |
| 70 | 15 |
| 95 | 30 |
| 97 | 45 |
| 99 | 60 |

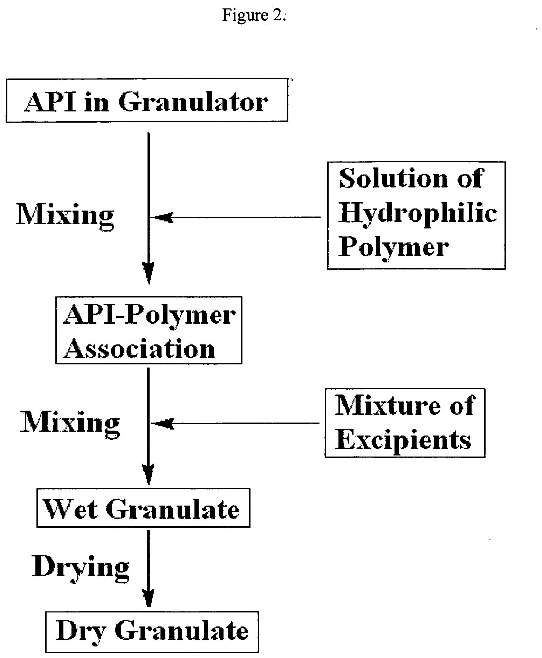

The present invention also relates to a process for making a granulate for use in a pharmaceutical composition which is an oral solid dosage form. The process is outlined in Figure 2. The process comprises the steps of: (a) forming a slurry of drug dispersion ingredients by combining an API having poor aqueous solubility with a solution or suspension of one or more pharmaceutically acceptable hydrophilic polymers; (b) forming a wet granulate by combining the granulation ingredients with a mixture of drug dispersion ingredients; and (c) drying the wet granulate to form a dry granulate. In one embodiment, the step of (a) forming a slurry of drug dispersion ingredients by combining an API having poor aqueous solubility with a solution or suspension of one or more pharmaceutically acceptable hydrophilic polymers comprises: (1) dissolving or suspending at least one hydrophilic polymer in a solvent to form a solution, and (2) combining the solution or suspension formed in step (a) with at least one active pharmaceutical ingredient having a solubility in water of less than about 1 mg/ml to form a mixture and blending the mixture to form a slurry. In another embodiment, the step of (b) forming a wet granulate by combining the granulation ingredients with the mixture of drug dispersion ingredients comprises: (3) forming a mixture of at least one diluent and at least one disintegrant; (4) combining the slurry of the at least one active ingredient and the at least one hydrophilic polymer with the mixture formed in step (3), and (5) mixing the mixture of step (4) to form a wet granulate. The processes of combining and/or mixing can be by any mixing or dispersing means as is known in the art. For example, the ingredients can be combined using a twin-shell mixer of the Patterson-Kelly type, a planetary mixer of the Glen type, or a high shear/high intensity or high speed mixer of the Henschel, Lodige/Littleford, or Baker-Perkins types. Use of a low shear mixer is the preferred means of combining ingredients, especially when forming the slurry of the at least one API and the at least one hydrophilic polymer. The wet granulate can be dried, using methods that are well known to those in the art such as, for example, in a tray drier or fluidized bed drier.

The dry granulate may optionally be further processed to alter the particle size distribution of the granulate and to add additional ingredients, such as at least one lubricant, to the granulate. Processes for altering the distribution of particle sizes are well known in the art and include milling, screening and combinations thereof. For example, a Fitzpatrick mill with an appropriate size screen, such as, for example, a 0.5 mm screen can be suitable for use in this step.

A pharmaceutical composition in an oral dosage form, such as tablets or capsules, may be prepared using granulates described above. The dry granulate obtained by the methods described above can be used directly, or can be blended with one or more additional pharmaceutically acceptable excipients prior to use. In one embodiment, the granulate is blended with at least one lubricant prior to use, for example, prior to being compressed into tablets. The dry granulates can be further processed to change the particle size distribution to a desired distribution. The particle size distribution of the granules may be adjusted to affect the dissolution profile or the release rate profile of the active ingredient from the formula. The dry granules may also be blended or combined with one or more additional pharmaceutically acceptable excipients prior to use in the pharmaceutical formulation. These excipients can include excipients described above. In an embodiment, the dry granules are combined with one or more lubricants. The pharmaceutical formulation can further comprise at least one coating. One of ordinary skill in the art would recognize that coatings can be used for a variety of purposes, including providing stability to the dosage form, adjusting the release rate of the API from the dosage form, adjusting the disintegration rate of the dosage form, and providing identification information regarding the dosage form. Such a person would also recognize how to select and use such coatings to achieve the desired effects.

The present invention also relates to a granulate prepared by any of the processes described above.

In order to further illustrate the present invention and the advantages thereof, the following specific examples are given, it being understood that same are intended only as illustrative and in no way limitative. In said examples to follow, all parts and percentages are given by weight, unless otherwise indicated.

Examples 1-3 are representative examples of tablet formulations comprising bicalutamide as the API in granules. The core of the granules comprises bicalutamide as the active ingredient and povidone as the hydrophilic polymer, where the core was formed by a slurry of bicalutamide and an aqueous solution of povidone. Differing amounts of povidone are present in the core. The granules comprises the core in contact with the various excipients indicated in the table as granulation ingredients, where the diluent is lactose monohydrate and the disintegrant is sodium starch glycolate. The amounts of lactose monohydrate and sodium starch glycolate vary between the formulations. In Example 3, povidone is also present as an excipient with lactose monohydrate and sodium starch glycolate in contact with the core. The tablets further comprise a mixture of lubricants comprising colloidal silicon dioxide and a mixture of magnesium stearate/sodium lauryl sulfate which were added to the granulate.

**Table 1. Examples of Bicalutamide Tablet Formulations:**

| Ingredients | % w/w | | |
|---|---|---|---|
| | Example 1 | Example 2 | Example 3 |
| Drug Dispersion Ingredients: | | | |
| Povidone (PLASDONE® K29/32 or KOLLIDON ® 30) | 5.0 | 10.0 | 3.63 |
| Purified Water, USP¹ | (17.3) | (17.3) | (10.9) |
| Bicalutamide | 41.67 | 41.67 | 41.67 |

| Granulation Ingredients: | | | |
|---|---|---|---|
| Lactose Monohydrate | 42.58 | 37.58 | 41.58 |
| Sodium Starch Glycolate | 9.0 | 9.0 | 3.0 |
| Povidone (PLASDONE® K29/32 or KOLLIDON ® 30) | 0.0 | 0.0 | 8.37 |

| Lubricant Ingredients: | | | |
|---|---|---|---|
| Magnesium Stearate/Sodium Lauryl Sulfate (94/6) (STEAR-O-WET® M) | 1.25 | 1.25 | 1.25 |
| Colloidal Silicon Dioxide | 0.5 | 0.5 | 0.5 |
| Total | 100 | 100 | 100 |

| | | | |
|---|---|---|---|
| ¹ Removed during processing and not part of the final tablet weight | | | |

Each of these example tablets were prepared using granulates made by the reverse wet granulation process of the present invention. The granulates along with the added lubricants were compressed to form tablets.

### Example 4:

Tablets of bicalutamide were prepared by the process described above using the ingredients listed in Table 2, below, in the amounts shown.

**Table 2. Composition of Bicalutamide Tablets:**

| Ingredient Description | mg |
|---|---|
| Bicalutamide | 50.0 |
| Povidone, K29-32/30 | 12.0 |
| Lactose, monohydrate | 45.1 |
| Sodium Starch Glycolate (Explotab/Glycolys/Primojel) | 10.8 |
| Magnesium Stearate/Sodium Lauryl Sulfate (94:6) (Stear- | 1.50 |
| Colloidal Silicon Dioxide (Cab-O-Sil, M-5) | 0.60 |
| Total Weight of Tablet Before Coating | 120.0 |
| COATING: White Opadry II (Y-22-7719) | 5.0 |
| Total Weight of Coated Tablet | 125.0 |

### Example 5:

The API, bicalutamide, and the hydrophilic polymer, povidone, were combined by dissolving povidone (18.0 g) in purified water (62.4 g) to form a solution and adding the solution, with mixing, to a powder of bicalutamide (150 g) in the bowl of a granulator. The mixture was stirred until a dispersion in the form of a slurry was formed. The granulation ingredients, lactose monohydrate (153.3 g) and sodium starch glycolate (32.4 g), where combined in a separate blender with mixing to form a mixture of the granulation ingredients. The mixture of the granulation ingredients were added to the dispersion of povidone, water and bicalutamide with mixing. The mixing was continued until a wet granulate was obtained. The wet granulate was dried in an oven until the desired moisture level was obtained. The dried granulate was then passed through a Fitzmill. These granules (272.4 g) were blended with wettable blend of magnesium stearate/sodium lauryl sulfate (94/6) (3.47 g) (STEAR-O-WET® M produced by Mallinckrodt) and colloidal silicon dioxide (1.39 g) and the resulting blend was compressed into tablets.

### Examples 6 and 7:

A comparison of the dissolution of tablets made from granules formed using the standard wet granulation process (Example 6) was made with the tablets made from granules formed using the reverse wet granulation process (Example 7) generally described above in Example 5, using the composition described in Example 2. The dissolution of the tablets of Examples 6 and 7 were evaluated using the FDA recommended dissolution test using USP apparatus II (paddle) with 1000 ml of a 1% aqueous solution of sodium lauryl sulfate at 37° C with the paddle apparatus rotating at 50 rpm. The results of the tests are summarized in Table 3, below.

**Table 3. Comparison of Reverse Wet Granulation Process with Standard Wet Granulation Process for Bicalutamide Tablets:**

| Granulating Process | Standard Granulation (Example 6) | Reverse Granulation (Example 7) |
|---|---|---|
| Process Variables | | |
| % Water Added | 15 | 15 |
| Mixing Time (min)¹ | 3 | 3 |
| Dissolution | % Dissolved (n=3) | |
| 5 min | 30 | 27 |
| 15 min | 73 | 72 |
| 30 min | 93 | 96 |
| 45 min | 97 | 101 |
| 60 min | 99 | 103 |
| Granulation Endpoint | Sharp, narrow range | Wide range |
| Dissolution Observations | Uneven erosion with large dispersed particles | Uniform erosion with small dispersed particles |

| | | |
|---|---|---|
| ¹ The mixing time after addition of all material during granulation. | | |

The results of this test indicate that tablets produced using granules formed by the reverse wet granulation process have a wider granulation endpoint range those produced using granules formed by the standard one-step process. In addition, tablets produced using granules formed by the reverse wet granulation process eroded more uniformly than those produced using granules formed by the standard one-step process.

### Example 8:

A bioequivalence study was conducted to compare the bioequivalence of bicalutamide tablets comprising granules formed using the reverse wet granulation method with the bioequivalence of commercially available tablets produced using the conventional granulation process (CASODEX® 50 mg tablets). The test used healthy male human volunteers ate a standard FDA breakfast meal 30 minutes prior to administration of a single oral dose of 50 mg of bicalutamide. Blood samples were collected from the volunteers at various times up to approximately 504 hours after dosing. The concentration of bicalutamide in plasma was determined by HPLC/MS (high performance liquid chromatography with mass spectrometric detection). The results of the determination of the blood concentrations were used to calculate the following pharmacokinetic parameters: the maximum concentration in the blood (CPEAK); the time at which the maximum concentration was observed (TPEAK); the elimination rate constant (KEL); the area under the plasma concentration-time curve (AUCL); the area under the plasma concentration-time curve from zero to infinity (AUCI); and the elimination half-life (HALF).

| Parameter | A = Reverse Wet Granulation | B = Conventional Process | LSMEANS Ratio (A:B) | 90% Confidence Interval |
|---|---|---|---|---|
| AUCL | 174188.2 | 155593.1 | | |
| (ng·hr/mL) | (23.8%) | (23.9%) | 1.13 | 104.7% -121.5% |
| AUCI | 190825.3 | 170507.3 | 1.13 | 103.3% - 123.9% |
| (ng·hr/mL) | (30.3%) | (32.9%) | | |
| CPEAK (ng/mL) | 1110.3 | 1041.0 | 1.06 | 101.1% - 111.9% |
| | (16.5%) | (13.7%) | | |
| KEL (hr⁻¹) | 0.0057 | 0.0062 | --- | --- |
| | (29.1 %) | (28.9%) | | |
| HALF (hr) | 131.3 | 123.1 | --- | --- |
| | (24.15%). | (35.6%) | | |
| TPEAK (hr) | 9.00 | 6.36 | --- | --- |
| | (93.5%) | (68.2%) | | |

| | | | | |
|---|---|---|---|---|
| Values are arithmetic means | | | | |

These results indicate that tablets comprising granules produced by the reverse wet granulation process are bioequivalent to those produced by the conventional wet granulation process.

### Example 9:

A comparison of pharmacokinetic profiles, fasted and fed, in patients dosed with bicalutamide tablets made by the reverse wet granulation (test) process are given below. The methodology used was as described above in Example 8 except for the fasting versus fed states of the test subjects. In both fasted and fed states, a comparison was also made to the profile of commercially available tablets produced using the conventional (reference) granulation process (CASODEX® 50 mg tablets).

| Fasting | | | | |
|---|---|---|---|---|
| Parameter | Test (n = 50) | Reference (n = 49) | Ratio* | 90% C.I.** |
| AUC0-t (ng × hr/mL) | 145863.5 | 146808.9 | 0.99 | 92.8% - 106.5% |
| AUC∞ (ng × hr/mL) | 156603.6 | 156403.3 | 1.00 | 92.4% - 108.5 |
| Cₘₐₓ (ng/mL) | 799.3 | 844.3 | 0.95 | 89.8% - 99.9% |
| | | | | |

| Fed | | | | |
|---|---|---|---|---|
| Parameter | Test (n = 48) | Reference (n = 45) | Ratio* | 90% C.I.** |
| AUC0-t (ng × hr/mL) | 170233.2 | 151635.6 | 1.13 | 104.7% - 121.5% |
| AUC∞ (ng × hr/mL) | 184321.2 | 163689.2 | 1.13 | 103.3% - 123.9% |
| Cₘₐₓ (ng/mL) | 1096.2 | 1032.0 | 1.06 | 101.1%-111.9% |

| | | | | |
|---|---|---|---|---|
| Values are Least Squares Geometric Means *Ratio (A/B) = e^{[LSMEAN of LNA - LSMEAN of LNB]} **Used Natural Log Transformed Parameter | | | | |

These results indicate that tablets comprising granules produced by the reverse wet granulation process are bioequivalent to those produced by the conventional wet granulation process when used to treat either patients having fasted before treatment or patients having eaten before treatment.

## Claims

1. A granule for a pharmaceutical composition, comprising a core which comprises at least one active pharmaceutical ingredient selected from bicalutamide and fenofibrate intimately associated with at least one hydrophilic polymer,
wherein the granule is prepared from a slurry formed by: (a) combining the at least one active pharmaceutical ingredient with a solution or suspension of the least one hydrophilic polymer; (b) combining the slurry with at least one excipient.

2. The granule of claim 1, wherein the at least one excipient comprises at least one diluent and at least one disintegrant, optionally further comprising a lubricant.

3. The granule of claim 1, where the at least one hydrophilic polymer is selected from the group consisting of polyvinyl pyrrolidone, hydroxypropyl methylcellulose, hydroxypropyl cellulose, polyethylene glycol, hydroxyethyl cellulose, polyethylene oxide, carbomer, polyvinyl alcohol, and/or mixtures thereof.

4. The granule of claim 2, where the at least one diluent is selected from the group consisting of lactose monohydrate, microcrystalline cellulose, calcium phosphate dibasic, sucrose, mannitol, starch, pregelatinized starch, lactose, sorbitol, glucose, fructose, galactose, maltose, isomaltose, aluminum oxide, bentonite, powdered cellulose, kaolin, magnesium carbonate, saponite, and mixtures thereof.

5. The granule of claim 2, where the at least one disintegrant is selected from the group consisting of crospovidone, croscarmellose sodium, sodium starch glycolate, and mixtures thereof.

6. The granule of claim 1, where the granulate further comprises at least one lubricant selected from the group consisting of magnesium stearate, sodium lauryl sulfate, colloidal silicon dioxide, calcium stearate, magnesium lauryl sulfate, potassium benzoate, sodium benzoate, talc, zinc stearate, sodium stearyl fumarate and mixtures thereof.

7. The granule of claim 2, where the at least one diluent is lactose monohydrate, the disintegrant is sodium starch glycolate, and the lubricant is magnesium stearate/sodium lauryl sulfate and colloidal silicon dioxide.

8. A pharmaceutical dosage form, comprising the granulate of claims 1-7.

9. A pharmaceutical tablet comprising granules wherein individual granules have a core of bicalutamide intimately associated with povidone, the individual granules further comprising a diluent, a disintegrant and a lubricant,
wherein the granules are prepared by (a) forming a slurry of drug dispersion ingredients by combining bicalutamide with a solution or suspension of povidone; (b) forming wet granules by combining the diluent, disintegrant and lubricant with the slurry; and (c) drying the wet granules to form dry granules.

10. A process for making a granulate, comprising:
(a) forming a slurry of drug dispersion ingredients by combining an active pharmaceutical ingredient selected from bicalutamide and fenofibrate with a solution or suspension of one or more pharmaceutically acceptable hydrophilic polymers;
(b) forming a wet granulate by combining the granulation ingredients with the mixture of drug dispersion ingredients; and
(c) drying the wet granulate to form a dry granulate
wherein the dry granulate comprises a core which comprises the active pharmaceutical ingredient intimately associated with the one or more pharmaceutically acceptable hydrophilic polymers.

11. A process for making a granulate, comprising:
(a) dissolving or suspending at least one hydrophilic polymer in a solvent to form a solution or suspension, respectively;
(b) combining the solution formed in step (a) with an active pharmaceutical ingredient selected from bicalutamide and fenofibrate to form a mixture and blending the mixture to form a slurry;
(c) forming a mixture of at least one diluent and at least one disintegrant;
(d) combining the mixture formed in step (b) with the mixture formed in step (c);
(e) mixing the mixture of step (d) to form a wet granulate; and
(f) drying the wet granulate to obtain a dry granulate
wherein the dry granulate comprises a core which comprises the active pharmaceutical ingredient intimately associated with the one or more pharmaceutically acceptable hydrophilic polymers.

12. The process of claim 10 or 11, further comprising the step of blending at least one lubricant and/or other excipient with the dry granulate.

13. The process of claim 10 or 11, further comprising the step of processing the dry granulate to modify the particle size distribution of the dry granulate.

14. A granulate formed by the process of claim 10-13.

15. A process for making a pharmaceutical dosage form in the form of a tablet or capsule, wherein when the pharmaceutical dosage form is a tablet, the process comprises compressing the granulate of claim 1 into tablets; and wherein when the pharmaceutical dosage form is a capsule, the process comprises filling a capsule shell with the granulate for pharmaceutical composition of claim 1 to obtain the capsule, said process optionally comprising the step of including one or more additional pharmaceutically acceptable excipients, wherein the pharmaceutically acceptable excipients are optionally selected from the group consisting of diluents, disintegrants, binders, wetting agents, lubricants, glidants, coloring agents and flavoring agents.

## Patentansprüche

1. Granulat für eine pharmazeutische Zusammensetzung, umfassend einen Kern, der wenigstens einen pharmazeutischen Wirkstoff umfasst, der aus Bicalutamid und Fenofibrat ausgewählt ist, das eng mit wenigstens einem hydrophilen Polymer verknüpft ist, wobei das Granulat aus einer Aufschlämmung hergestellt wird, die durch Folgendes ausgebildet wird: (a) Kombinieren des wenigstens einen pharmazeutischen Wirkstoffs mit einer Lösung oder Suspension des wenigstens einen hydrophilen Polymers; (b) Kombinieren der Aufschlämmung mit wenigstens einem Hilfsstoff.

2. Granulat nach Anspruch 1, wobei der wenigstens eine Hilfsstoff wenigstens ein Verdünnungsmittel und wenigstens ein Sprengmittel umfasst, optional ferner ein Schmiermittel umfassend.

3. Granulat nach Anspruch 1, wobei das wenigstens eine hydrophile Polymer aus der Gruppe ausgewählt ist, bestehend aus Polyvinylpyrrolidon, Hydroxypropylmethylcellulose, Hydroxypropylcellulose, Polyethylenglykol, Hydroxyethylcellulose, Polyethylenoxid, Carbomer, Polyvinylalkohol und/oder Mischungen davon.

4. Granulat nach Anspruch 2, wobei das wenigstens eine Verdünnungsmittel aus der Gruppe ausgewählt ist, bestehend aus Lactosemonohydrat, mikrokristalliner Cellulose, zweibasischem Calciumphosphat, Saccharose, Mannit, Stärke, vorgelatinierter Stärke, Lactose, Sorbit, Glucose, Fructose, Galactose, Maltose, Isomaltose, Aluminiumoxid, Bentonit, pulverisierter Cellulose, Kaolin, Magnesiumcarbonat, Saponit und Mischungen davon.

5. Granulat nach Anspruch 2, wobei das wenigstens eine Sprengmittel aus der Gruppe ausgewählt ist, bestehend aus Crospovidon, Croscarmellosenatrium, Natriumstärkeglykolat und Mischungen davon.

6. Granulat nach Anspruch 1, wobei das Granulat ferner wenigstens ein Schmiermittel umfasst, das aus der Gruppe ausgewählt ist, bestehend aus Magnesiumstearat, Natriumlaurylsulfat, kolloidalem Siliciumdioxid, Calciumstearat, Magnesiumlaurylsulfat, Kaliumbenzoat, Natriumbenzoat, Talk, Zinkstearat, Natriumstearylfumarat und Mischungen davon.

7. Granulat nach Anspruch 2, wobei das wenigstens eine Verdünnungsmittel Lactosemonohydrat ist, das Sprengmittel Natriumstärkeglykolat ist und das Schmiermittel Magnesiumstearat/Natriumlaurylsulfat und kolloidales Siliciumdioxid ist.

8. Pharmazeutische Dosierungsform, umfassend das Granulat nach Ansprüchen 1 bis 7.

9. Pharmazeutische Tablette, umfassend Granulate, wobei einzelne Granulate einen Kern aus Bicalutamid aufweisen, der eng mit Povidon verknüpft ist, wobei die einzelnen Granulate ferner ein Verdünnungsmittel, ein Sprengmittel und ein Schmiermittel umfassen, wobei die Granulate durch (a) das Ausbilden einer Aufschlämmung von Arzneimitteldispersionsbestandteilen durch das Kombinieren von Bicalutamid mit einer Lösung oder Suspension von Povidon; (b) das Ausbilden feuchter Granulate durch das Kombinieren des Verdünnungsmittels, des Sprengmittels und des Schmiermittels mit der Aufschlämmung; und (c) das Trocknen der feuchten Granulate zum Ausbilden trockener Granulate hergestellt wird.

10. Verfahren zum Herstellen eines Granulats, Folgendes umfassend:
(a) Ausbilden einer Aufschlämmung von Arzneimitteldispersionsbestandteilen durch das Kombinieren eines aus Bicalutamid und Fenofibrat ausgewählten pharmazeutischen Wirkstoffs mit einer Lösung oder Suspension eines oder mehrerer pharmazeutisch unbedenklicher hydrophiler Polymere;
(b) Ausbilden eines feuchten Granulats durch das Kombinieren der Granulationsbestandteile mit der Mischung von Arzneimitteldispersionsbestandteilen; und
(c) Trocknen des feuchten Granulats zum Ausbilden eines trockenen Granulats,
wobei das trockene Granulat einen Kern umfasst, der den pharmazeutischen Wirkstoff umfasst, der eng mit dem einen oder den mehreren pharmazeutisch unbedenklichen hydrophilen Polymeren verknüpft ist.

11. Verfahren zum Herstellen eines Granulats, Folgendes umfassend:
(a) Lösen oder Suspendieren wenigstens eines hydrophilen Polymers in einem Lösungsmittel, um eine Lösung beziehungsweise eine Suspension auszubilden;
(b) Kombinieren der in Schritt (a) ausgebildeten Lösung mit einem pharmazeutischen Wirkstoff, der aus Bicalutamid und Fenofibrat ausgebildet ist, um eine Mischung auszubilden, und Mischen der Mischung, um eine Aufschlämmung auszubilden;
(c) Ausbilden einer Mischung aus wenigstens einem Verdünnungsmittel und wenigstens einem Sprengmittel;
(d) Kombinieren der in Schritt (b) ausgebildeten Mischung mit der in Schritt (c) ausgebildeten Mischung;
(e) Mischen der Mischung aus Schritt (d), um ein feuchtes Granulat auszubilden; und
(f) Trocknen des feuchten Granulats, um ein trockenes Granulat zu erhalten,
wobei das trockene Granulat einen Kern umfasst, der den pharmazeutischen Wirkstoff umfasst, der eng mit dem einen oder den mehreren pharmazeutisch unbedenklichen hydrophilen Polymeren verknüpft ist.

12. Verfahren nach Anspruch 10 oder 11, ferner umfassend den Schritt des Mischens wenigstens eines Schmiermittels und/oder eines anderen Hilfsstoffs mit dem trockenen Granulat.

13. Verfahren nach Anspruch 10 oder 11, ferner umfassend den Schritt des Verarbeitens des trockenen Granulats zum Modifizieren der Teilchengrößenverteilung des trockenen Granulats.

14. Granulat, ausgebildet nach dem Verfahren nach Anspruch 10 bis 13.

15. Verfahren zum Herstellen einer pharmazeutischen Dosierungsform in der Form einer Tablette oder einer Kapsel, wobei, wenn die pharmazeutische Dosierungsform eine Tablette ist, das Verfahren das Komprimieren des Granulats nach Anspruch 1 zu Tabletten umfasst; und wobei, wenn die pharmazeutische Dosierungsform eine Kapsel ist, das Verfahren das Füllen einer Kapselhülle mit dem Granulat für die pharmazeutische Zusammensetzung nach Anspruch 1 umfasst, um die Kapsel zu erhalten, wobei das Verfahren optional den Schritt des Einschließens eines oder mehrerer zusätzlicher pharmazeutisch unbedenklicher Hilfsstoffe umfasst, wobei die pharmazeutisch unbedenklichen Hilfsstoffe optional aus der Gruppe ausgewählt werden, bestehend aus Verdünnungsmitteln, Sprengmitteln, Bindemitteln, Netzmitteln, Schmiermitteln, Gleitmitteln, Farbstoffen und Aromastoffen.

## Revendications

1. Granule pour une composition pharmaceutique, comprenant un noyau qui comprend au moins un ingrédient pharmaceutiquement actif choisi parmi le bicalutamide et le fénofibrate, intimement associé avec au moins un polymère hydrophile,
le granule étant préparé à partir d'une bouillie formée par : (a) combinaison du ou des ingrédients pharmaceutiquement actifs avec une solution ou une suspension du ou des polymères hydrophiles ; (b) combinaison de la bouillie avec au moins un excipient.

2. Granule de la revendication 1, dans lequel le ou les excipients comprennent au moins un diluant et au moins un désintégrant, comprenant en outre facultativement un lubrifiant.

3. Granule de la revendication 1, dans lequel le ou les polymères hydrophiles sont choisis parmi le groupe constitué de polyvinyl pyrrolidone, hydroxypropyl méthylcellulose, hydroxypropyl cellulose, polyéthylène glycol, hydroxyéthyl cellulose, oxyde de polyéthylène, carbomère, alcool polyvinylique et/ou de mélanges de ceux-ci.

4. Granule de la revendication 2, dans lequel le ou les diluants sont choisis parmi le groupe constitué de lactose monohydraté, cellulose microcristalline, phosphate de calcium dibasique, saccharose, mannitol, amidon, amidon prégélatinisé, lactose, sorbitol, glucose, fructose, galactose, maltose, isomaltose, oxyde d'aluminium, bentonite, cellulose pulvérisée, kaolin, carbonate de magnésium, saponite, et des mélanges de ceux-ci.

5. Granule de la revendication 2, dans lequel le ou les désintégrants sont choisis parmi le groupe constitué de crospovidone, croscarmellose sodium, glycolate d'amidon sodique, et de mélanges de ceux-ci.

6. Granule de la revendication 1, dans lequel le granulé comprend en outre au moins un lubrifiant choisi parmi le groupe constitué de stéarate de magnésium, laurylsulfate de sodium, dioxyde de silicone colloïdal, stéarate de calcium, laurylsulfate de magnésium, benzoate de potassium, benzoate de sodium, talc, stéarate de zinc, stéarylfumarate de sodium et de mélanges de ceux-ci.

7. Granule de la revendication 2, dans lequel le ou les diluants sont le lactose monohydraté, le désintégrant est le glycolate d'amidon sodique, et le lubrifiant est le stéarate de magnésium/laurylsulfate de sodium et le dioxyde de silicone colloïdal.

8. Forme pharmaceutique, comprenant le granulé des revendications 1 à 7.

9. Comprimé pharmaceutique comprenant des granules, dans lequel les granules individuels ont un noyau de bicalutamide intimement associé avec la povidone, les granules individuels comprenant en outre un diluant, un désintégrant et un lubrifiant, dans lequel les granules sont préparés par (a) formation d'une bouillie d'ingrédients de dispersion de médicament en combinant le bicalutamide avec une solution ou une suspension de povidone ; (b) formation de granules humides par combinaison du diluant, du désintégrant et du lubrifiant avec la bouillie ; et (c) séchage des granules humides pour former des granules secs.

10. Procédé de préparation d'un granulé, comprenant :
(a) la formation d'une bouillie d'ingrédients de dispersion de médicament par combinaison d'un ingrédient pharmaceutique actif choisi parmi le bicalutamide et le fénofibrate avec une solution ou une suspension d'un ou plusieurs polymères hydrophiles pharmaceutiquement acceptables ;
(b) la formation d'un granulé humide par combinaison des ingrédients de granulation avec le mélange des ingrédients de dispersion de médicament ; et
(c) le séchage du granulé humide pour former un granulé sec
le granulé sec comprenant un noyau qui comprend l'ingrédient pharmaceutique actif intimement associé avec le ou les polymères hydrophiles pharmaceutiquement acceptables.

11. Procédé de préparation d'un granulé, comprenant :
(a) la dissolution ou la mise en suspension d'au moins un polymère hydrophile dans un solvant pour former une solution ou une suspension, respectivement ;
(b) la combinaison de la solution formée à l'étape (a) avec un ingrédient pharmaceutique actif choisi parmi le bicalutamide et le fénofibrate pour former un mélange et l'homogénéisation du mélange pour former une bouillie ;
(c) la formation d'un mélange d'au moins un diluant et d'au moins un désintégrant ;
(d) la combinaison du mélange formé à l'étape (b) avec le mélange formé à l'étape (c) ;
(e) l'homogénéisation du mélange de l'étape (d) pour former un granulé humide ; et
(f) le séchage du granulé humide pour obtenir un granulé sec
le granulé sec comportant un noyau qui comprend l'ingrédient pharmaceutique actif intimement associé avec le ou les polymères hydrophiles pharmaceutiquement acceptables.

12. Procédé de la revendication 10 ou 11, comprenant en outre l'étape de mélange d'au moins un lubrifiant et/ou d'un autre excipient avec le granulé sec.

13. Procédé de la revendication 10 ou 11, comprenant en outre l'étape de traitement du granulé sec pour modifier la distribution de la taille des particules du granulé sec.

14. Granulé formé par le procédé des revendications 10 à 13.

15. Procédé de préparation d'une forme pharmaceutique sous la forme d'un comprimé ou d'une capsule, le procédé comprenant la compression du granulé de la revendication 1 en comprimés, lorsque la forme pharmaceutique est un comprimé ; et le procédé comprenant le remplissage d'une enveloppe de capsule avec le granulé pour la composition pharmaceutique de la revendication 1 pour obtenir la capsule, lorsque la forme pharmaceutique est une capsule, ledit procédé comprenant facultativement l'étape consistant à inclure un ou plusieurs excipients pharmaceutiquement acceptables supplémentaires, dans lequel les excipients pharmaceutiquement acceptables sont facultativement choisis parmi le groupe constitué de diluants, désintégrants, liants, agents mouillants, lubrifiants, agents de glissement, agents colorants et d'agents aromatisants.
